# EUROPEAN PATENT APPLICATION

(11) **EP 0 875 210 A1**
(43) Date of publication of application: **04.11.1998**
(21) Application number: 98107326.5
(22) Date of filing: 22.04.1998
(51) Int. Cl.: A61B 19/02, B65D 55/02

(54) **Safety container for medical waste**

(30) Priority: 30.04.1997 IT MI971023
(71) Applicant: D.D.S. Sanificazione S.r.l., 22068 Monticello, (Province of Lecco) (IT)
(72) Inventor: Riva, Gianluca, 22068 Monticello (IT); Zoia, Tiziano,, 20055 Renate (IT); Cambiaghi, Alberto,, 20058 Villasanta (IT)
(74) Representative: Modiano, Guido, Dr.-Ing.

(57) **Abstract**

A container for sanitary and liquid waste, particularly suitable in the hospital environment and the like, for the disposal of potentially hazardous waste, comprising a bin (2) which is meant to accommodate the waste, and a lid (3) associable therewith; the lid (3) has locking closure means (9) arranged at lateral strips (7) of the lid and suitable to engage corresponding seats (8) formed on the upper edge of the bin in order to lock the lid to the bin; the locking closure means are provided so as to break when the container is opened in order to clearly indicate that it has been opened after closure for transfer to a disposal facility.

## Description

The present invention relates to a container for sanitary waste and liquid waste with high utilization safety, particularly suitable for use in the hospital field for the disposal of waste which may be potentially infected or which in any case must not come into direct contact with hospital personnel.

It is known that in hospital environments the problem is strongly felt of disposing of waste of sanitary origin, particularly as regards potentially infected waste, which is normally present in a hospital.

The ever-increasing diffusion of diseases caused by contagion with infected blood has in fact emphasized the need to provide for safe disposal of waste and forced the setting of standards for regulating said disposal.

In particular, waste collection containers are currently used in the hospital sector which are made of steel and are washable and thus reusable or are made of cardboard and are of the single-use type, since it is not possible to wash them and then sterilize them.

Currently applicable statutory provisions prescribe that a container for hazardous (potentially infected) waste must have measures to prevent its accidental or fraudulent opening after closure performed before transfer to a disposal center.

In this regard, therefore, conventional containers have closure systems which do not allow to reopen the container once it has been closed without damaging the closure mechanism and therefore at the same time do not allow to reuse said container, causing a drawback as to costs.

Another drawback of conventional single-use containers made of cardboard is the fact that they can be easily perforated by the waste contained therein, with evident danger of contact with infected materials on the part of individuals who handle said containers.

The aim of the present invention is to provide a container for sanitary and liquid waste, particularly of the hospital type and the like, which can be used both as a single-use container and as a reusable container, in compliance with applicable statutory provisions regarding the disposal of hazardous waste.

Within the scope of this aim, an object of the present invention is to provide a container for sanitary and liquid waste, particularly of the hospital type and the like, which can have either single-use or reusable closure means.

Another object of the present invention is to provide a container for sanitary and liquid waste, particularly of the hospital type and the like, having a hermetic seal.

Another object of the present invention is to provide a container for sanitary and liquid waste which is made of a material which is absolutely perforation-proof.

Another object of the present invention is to provide a container for sanitary and liquid waste which is highly reliable, relatively easy to manufacture and at competitive costs.

This aim, these objects and others which will become apparent hereinafter are achieved by a container for sanitary and liquid waste, particularly suitable in the hospital environment and the like, for the disposal of potentially hazardous waste, comprising a bin which is meant to accommodate the waste, and a lid associable therewith, characterized in that said lid has locking closure means arranged at lateral strips of said lid and suitable to engage corresponding seats formed on the upper edge of said bin in order to lock said lid to the bin, said locking closure means being provided so as to break when the container is opened in order to clearly indicate that it has been opened after closure for transfer to a disposal facility.

Further characteristics and advantages of the invention will become apparent from the following detailed description of a preferred but not exclusive embodiment of the device according to the invention, illustrated only by way of non-limitative example in the accompanying drawings, wherein:

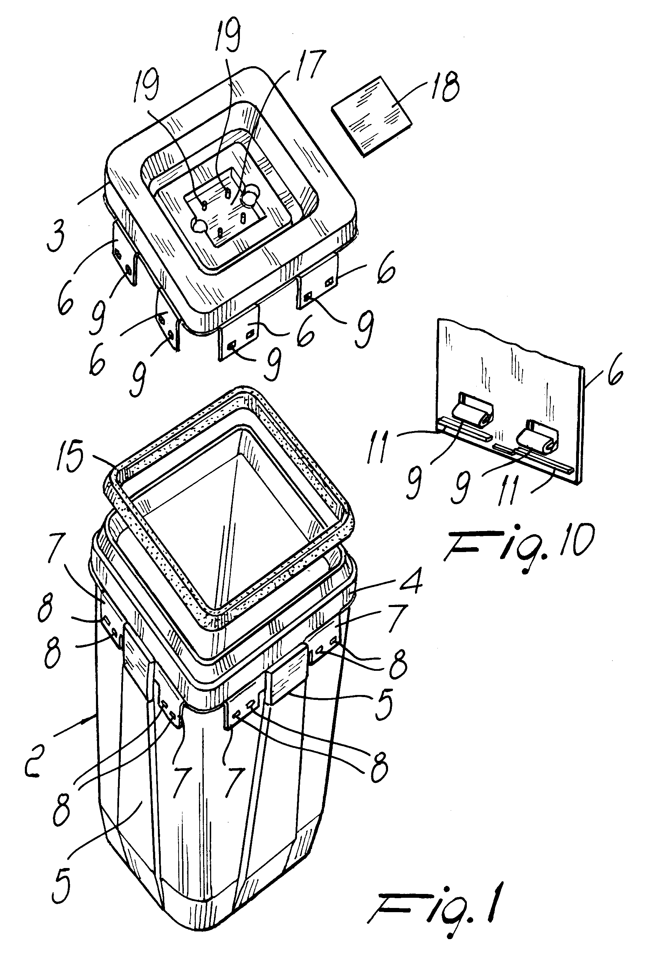

Figure 1 is an exploded perspective view of the container according to the present invention;
Figure 2 is a front elevation view of the container according to the present invention;

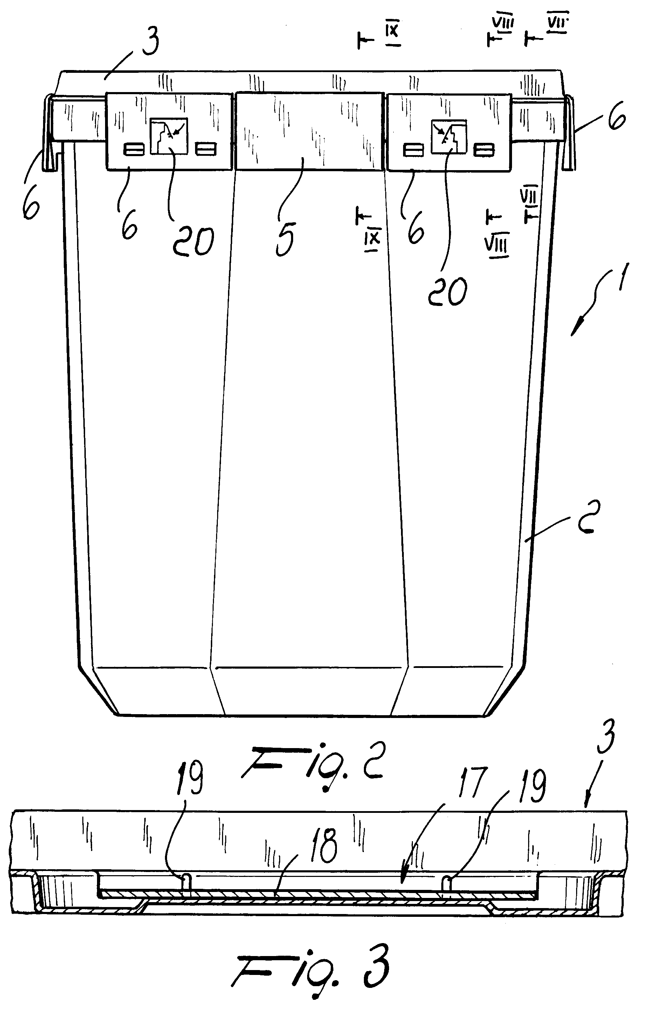

Figure 3 is a transverse sectional view of the container according to the invention, shown in Figure 2;

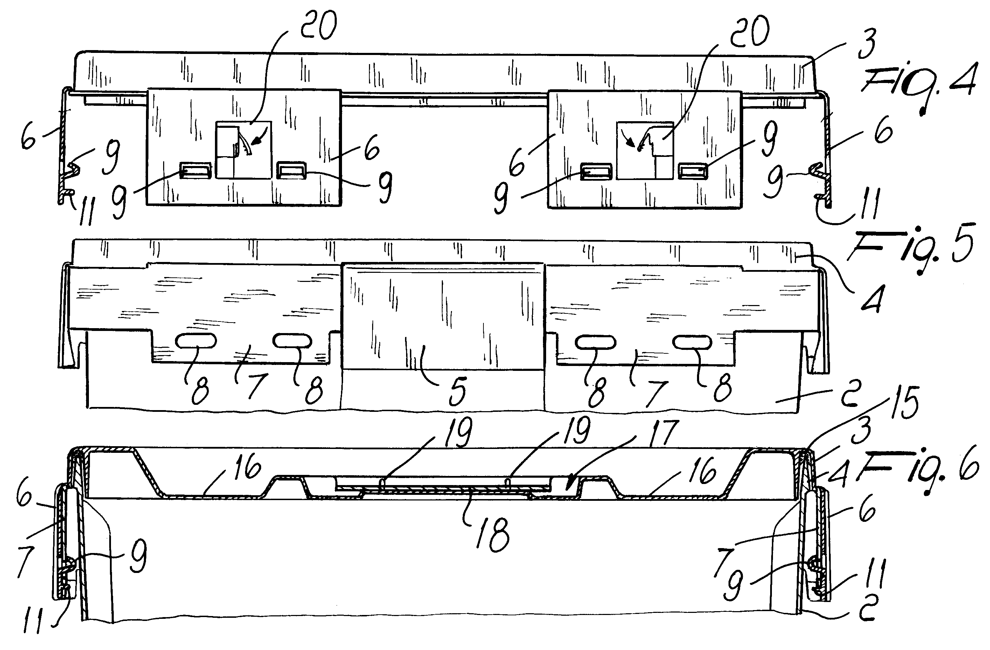

Figure 4 is a partially sectional lateral elevation view of the lid of the container according to the present invention;
Figure 5 is a partial lateral elevation view of the upper edge of the container according to the present invention without the corresponding lid;
Figure 6 is a partial sectional lateral elevation view of the container shown in Figure 5, with the lid applied;

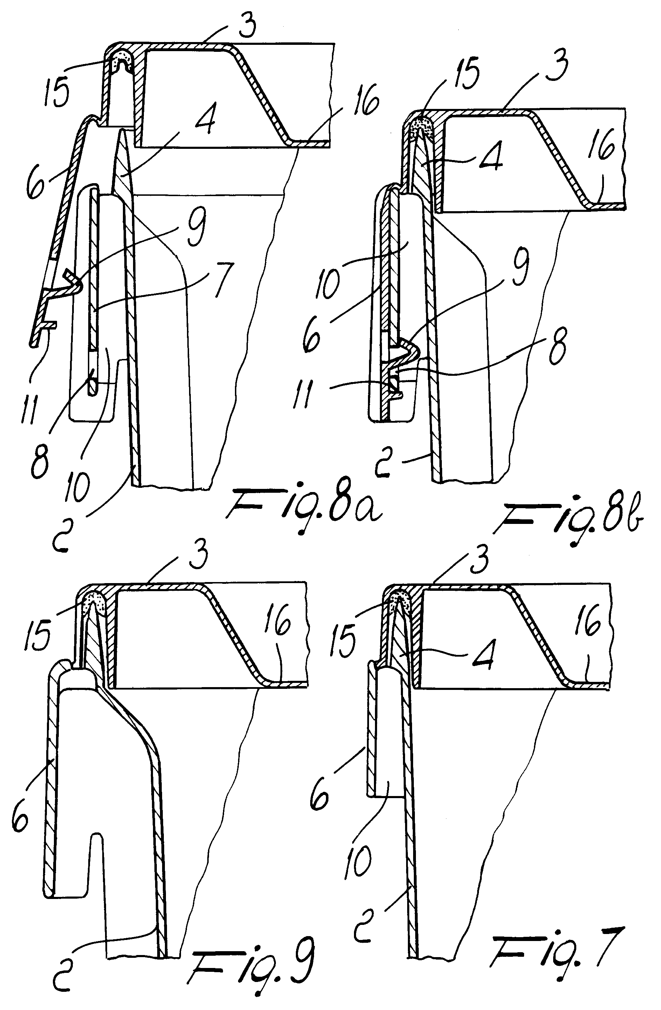

Figure 7 is a partial sectional view, taken along the plane VII-VII of Figure 2;
Figure 8a is a partial sectional view, taken along the plane VIII-VIII of Figure 2, of the container with the lid in the position before closure;
Figure 8b is a partial sectional view, similar to Figure 8a, of the container with the lid in the closure position;
Figure 9 is a partial sectional view, taken along the plane IX-IX of Figure 2; and
Figure 10 is a detailed perspective view of a detail of the closure system of the container according to the present invention.

With reference to the above figures, the container according to the present invention, generally designated by the reference numeral 1, comprises a bin 2 suitable to contain a bag for collecting sanitary and liquid waste.

The bin 2 is preferably shaped like a truncated pyramid in which the larger end face is directed upward and constitutes the mouth of the bin.

The bin 2 has a removable lid 3 meant to ensure, in the closed position, the tightness of the container when it must be transferred to a disposal facility.

The bin 2 is provided with a lateral rim 4 that runs along the upper edge of the bin and constitutes both a handle for lifting and generally moving the bin and an element to allow engagement of the lid 3, as described hereinafter.

A central ridge 5 runs longitudinally with respect to the bin 2 along each side thereof from the base edge of the bin to the rim 4 and blends therewith.

In a position which is adjacent to the rim 4 there are provided strip-like elements 7 which protrude from the rim 4 and have slots 8 which constitute engagement seats for engagement elements formed on corresponding lateral strips 6 which protrude from the lower edge of the lid 3.

Conveniently, the engagement elements are constituted for example by V-shaped teeth 9 which are meant to engage the slots 8 to lock the lid 3 on the bin 2 so as to lock and retain the lid 3 and therefore prevent its accidental or fraudulent removal after the container 1 has been closed.

The upper edge of the bin 2 of the container 1 is shaped so as to form a channel 10 in which one wall is constituted by the wall of the bin 2 and another wall is formed by the rim 4 that protrudes along the perimeter of the mouth of the bin 2 of the container. In this manner, the bin 2 of the container has a sort of perimetric handle which allows easy grip for moving it.

The lid 3 is in turn provided, at its lower edge, with a contour which is suitable to couple to the upper edge of the bin 2 of the container.

The strips 7 of the lid 3 also have, at their lower end, teeth 11 which are suitable to engage by friction the lower end of the strip-like element 7 of the bin 2, so as to further increase the retention ensured by the teeth 9.

Advantageously, the V-shaped teeth 9 are provided so that they break when the container 1 is to be reopened after closure for transfer to a disposal facility.

Figure 8b illustrates the teeth 9 in the position in which the lid 3 is closed and the teeth are engaged in the slots 8 thereof.

If the lid must be reused, the teeth 9 are not provided and closure is performed by means of the secondary teeth 11, which engage the lower end of the strip-like element 7. Thus, with the same container according to the invention a model is available which is useful both as a single-use container and as a reusable container.

After breaking the V-shaped teeth 9, it is also possible to reuse the lid so as to have both evidence that the container 1 has been opened and a container which can be reused without necessarily replacing the broken teeth 9, i.e., without having to replace the lid.

Regardless of whether the container 1 must be disposable or reusable, the teeth 9 can be provided either by molding them directly with each strip 6 of the lid 3 or as separate elements for subsequent insertion in the slots 8 when the lid is placed on the mouth of the container 1.

In any case, the clearly noticeable breakage of the teeth 9 constitutes certain proof that the container 1 has been opened, as prescribed by applicable statutory provisions.

Another characteristic of the lid 3 is the presence of regions for stiffening the lid, designated by the reference numeral 16, which are constituted by four recessed regions of the lid that are arranged at its four corners.

The lid 3 also has a recessed central region 17 which is suitable to accommodate a tag 18 for identifying the container 1 when closed and ready for transfer to a disposal facility. The tag 18 is retained by pins 19 and can easily be removed if the bin 2 is reused.

As a further characteristic, the lateral strips 6 of the lid 3 have markings 20 which explain how to close the container by engaging the teeth 9 in the slots 8.

The bin 2 of the container 1 and its lid 3 are both made of a rigid material which cannot be perforated by the waste meant to be contained in the container. Preferably, therefore, the container 1 is made of plastics, such as polyethylene or polypropylene.

The version made of polypropylene can be placed in an autoclave for sterilization.

A sealing gasket 15 is accommodated inside the upper edge of the lid 3 so as to abut, when closed, against the upper edge of the bin 2 so as to produce a hermetic closure of the container 1, which is thus suitable to also contain liquid waste.

Preferably, the gasket 15, which is made of rubber, is molded and coextruded with the lid 3. Accordingly, the rubber of the gasket 15 is also injected during the injection of the polymer for molding the container 1. The gasket 15 can be sterilized without damage.

The containers 1 thus manufactured are perfectly stackable and arrangeable on pallets for transport.

In practice it has been observed that the container according to the invention fully achieves the intended aim and objects, since it can be used both as single-use container and as reusable container. In the first case, the closure means are designed to break when the container is opened, so as to clearly indicate if it has been opened without authorization.

In the second case, instead, the closure means can be as described above or be such that they do not break during opening: in this second case, in order to comply with safety standards for the transport and disposal of hazardous waste, it is necessary to provide second closure means, this time of a kind meant to break when the container is opened.

The device thus conceived is susceptible of numerous modifications and variations, all of which are within the scope of the inventive concept; all the details may also be replaced with other technically equivalent elements.

In practice, the materials employed, so long as they are compatible with the specific use, as well as the dimensions, may be any according to requirements and to the state of the art.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A container for sanitary and liquid waste, particularly suitable in the hospital environment and the like, for the disposal of potentially hazardous waste, comprising a bin which is meant to accommodate the waste, and a lid associable therewith, characterized in that said lid has locking closure means which are arranged at lateral strips of said lid and are suitable to engage corresponding seats formed on the upper edge of said bin in order to lock said lid to the bin, said locking closure means being provided so as to break when the container is opened in order to clearly indicate that it has been opened after closure for transfer to a disposal facility.

2. A container according to claim 1, characterized in that said lid and said bin are made of plastics.

3. A container according to claim 2, characterized in that said bin and said container are made of polyethylene or polypropylene.

4. A container according to claim 1, characterized in that said locking closure means comprise at least one retention tooth suitable to engage one of said corresponding seats, said tooth being provided so that it breaks when the container is opened in order to clearly indicate that opening has occurred after closure for transport.

5. A container according to claim 4, characterized in that said locking closure means comprise at least one retention tooth for each lateral strip of a pair of mutually opposite sides of said lid.

6. A container according to claim 5, characterized in that said at least one retention tooth is V-shaped.

7. A container according to claim 5, characterized in that it has additional locking closure means arranged adjacent to said tooth, said additional locking closure means being provided so that they do not break when said container is opened.

8. A container according to claim 1, characterized in that it comprises a sealing element arranged between said lid and the upper edge of the bin to which said lid engages, said sealing element being coextruded with said lid.

9. A container according to claim 1, characterized in that said lid has reinforcement elements on its upper surface, said elements being constituted by recesses which are monolithic with said lid.

10. A container according to claim 1, characterized in that said lid is provided, in an upward region, with a recessed portion which is suitable to accommodate a removable tag for identifying the container on which said lid is placed.
